# EUROPÄISCHE PATENTSCHRIFT

(11) **EP 1 576 924 B1**
(45) Veröffentlichungstag und Bekanntmachung des Hinweises auf die Patenterteilung: **22.08.2007**
(21) Anmeldenummer: 05013875.9
(22) Anmeldetag: 10.09.2003
(51) Int. Cl.: A61B 6/14, G03B 42/04

(54) **Halter für digitale Sensoren für die Zahnmedizin**
Holder for digital sensors in dentistry
Support de détecteur digitale médico-dentaire

(30) Priorität: 30.09.2002 CH 16322002
(43) Veröffentlichungstag der Anmeldung: 21.09.2005
(62) Teilanmeldung aus: 03405661.4
(73) Patentinhaber: KerrHawe S.A., 6934 Bioggio (CH)
(72) Erfinder: Kilcher, Beat, 6935 Bosco-Luganese (CH); Da Rold, Marco, 6947 Vaglio (CH)
(74) Vertreter: AMMANN PATENTANWÄLTE AG BERN

(56) Entgegenhaltungen:
- EP-A- 1 020 759
- DE-U1- 20 100 641
- US-A- 1 571 145
- US-A- 4 965 885
- US-A- 5 677 537
- US-B1- 6 343 875

## Beschreibung

Die vorliegende Erfindung bezieht sich auf einen Halter-Satz für digitale Sensoren für die Zahnmedizin.

Bis vor kurzem wurden die Röntgenaufnahmen auf Röntgenfilmen fixiert, die belichtet und später entwickelt wurden. Solche Röntgenfilme haben bestimmte, genormte Formate, so dass die Halter, bzw. deren Klemmteil zur Aufnahme der Röntgenfilme, normierte Grössen aufweisen. Probleme bei solchen Röntgenfilmhalterungen ergaben und ergeben sich in erster Linie daraus, die Aufnahme von verschiedenen Zahnteilen, d.h. die Aufnahme von Bissflügel sowie anderen Teilen der Zähne mit möglichst wenig verschiedenen Haltern durchführen zu können, wobei die Filmformate meist entweder quer oder längs eingeklemmt werden.

In neuerer Zeit wurde eine neue Abbildungstechnik entwickelt, indem digitale Bildsensoren über Kabel direkt an einen Computer angeschlossen werden, um die Röntgenaufnahmen direkt darauf zu visualisieren oder zu speichern.

Im Gegensatz zu den Röntgenfilmen besitzen die verschiedenen Produkte von digitalen Sensoren verschiedene Dimensionen, sowohl in der Länge und Breite als auch in der Dicke. Hinzu kommt, dass solche Sensoren ein Kabel aufweisen, wodurch die lagerichtige Halterung solcher Sensoren problematisch ist.

Es sind bereits eine Anzahl von Sensorhaltern bekannt, so gemäss US-A-6 203 195, die einen Sensorhalter offenbart, bei dem ein Halterarm ausziehbar ist, um verschiedene Formate halten zu können. Obwohl hier bereits ein Ansatz vorliegt, verschiedene Formate zu verwenden, ist die universale Anwendung eines solchen Halters durch dessen Konstruktion eingeschränkt.

Aus der US-B1-6 343 875 ist ein Sensorhalter bekannt, dessen Sensorklemme auf einen Haltestab gesteckt werden kann. Die Sensorklemme ist derart ausgeführt, dass der Sensor einfach hineingedrückt wird. Daraus ist ersichtlich, dass die Sensorklemme nicht vorgesehen ist, eine grosse Vielfalt verschiedener Sensorformate aufzunehmen.

Aus der US-A-1 571 145 ist ein Röntgenfilmhalter bekannt, bei dem ein abgewinkelter Halterarm einen Röntgenfilm unten zwischen zwei Schenkeln hält und ein Keil auf einen der beiden Schenkel wirkt. Dabei wird der Röntgenfilm nur unten in einem schmalen Bereich gehalten. Dieser Filmhalter weist keine Mittel auf, um den Film bezüglich einer Röntgenröhre auszurichten und ist in diesem Sinne zwar für alle Zähne verwendbar, jedoch ohne genaues Arbeiten gewährleisten zu können.

Aus der US-A-4 965 885 ist ein Röntgenfilmhalter bekannt, der darauf gerichtet ist zu vermeiden, dass die scharfen Ecken des Film im Munde bemerkbar sind sowie dass genügend Platz vorhanden ist, um ein Wurzelkanal-Werkzeug einführen zu können. Für die Verwendung von Sensoren mit stark unterschiedlichen Formaten und den Zuleitungen ist dieser Halter nicht vorgesehen.

Es ist von diesem Stand der Technik ausgehend Aufgabe der vorliegenden Erfindung, einen Satz von Sensorenhaltern zu schaffen, die Röntgenaufnahmen mit sämtlichen sich auf dem Markt befindlichen digitalen Sensoren im Frontzähne- und Seitenbereich ermöglichen, d.h. im periapikalen Gewebe sowie horizontale und vertikale Bissflügelaufnahmen mit einerseits guter Ausrichtung und anderseits maximalem Komfort für den Patienten. Diese Aufgabe wird mit dem Haltersatz gemäss Patentanspruch 1 gelöst. Der abhängigen Anspruch 2 gibt eine bevorzugte Ausführungsform an.

Die Erfindung wird im Folgenden anhand von Zeichnungen eines Ausführungsbeispiels näher erläutert.
- Fig. 1: zeigt, teilweise aufgeschnitten, ein Ausführungsbeispiel eines Klemmteils für einen Halter für periapikale Aufnahmen,
- Fig. 2: zeigt das Klemmteil von Fig. 1 in Seitenansicht, und
- Fig. 3: zeigt ein Detail des Klemmteils von Fig. 1.

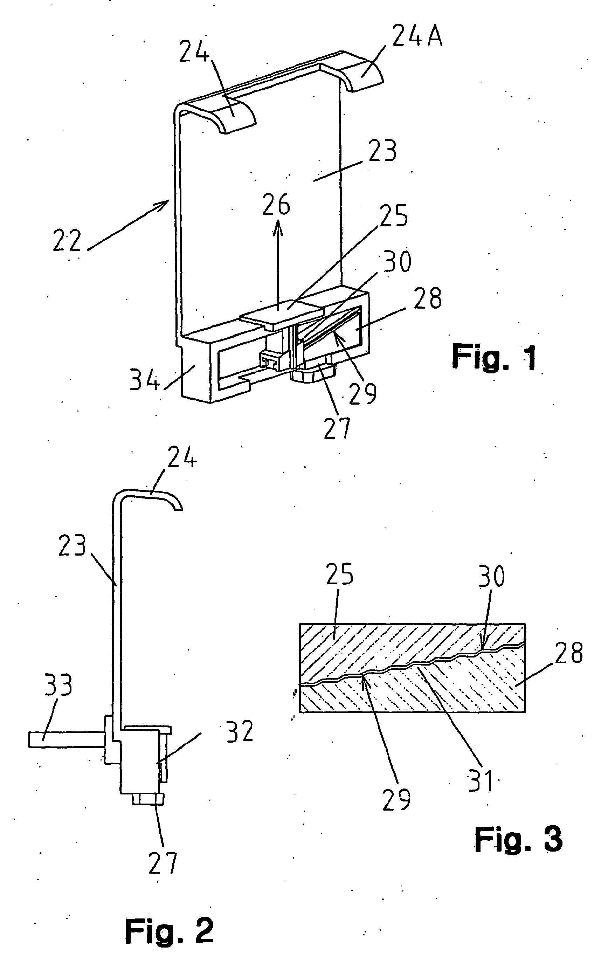

Aus den Figuren 1 - 3 ist ein Ausführungsbeispiel eines periapikalen Sensorhalters für posterior Zähne (Seitenzähne) dargestellt, wobei der eingelegte Sensor von unten nach oben geklemmt und nicht seitlich erfasst wird.

Die Sensorklemme enthält eine Wand 23, an der oben zwei Greifer 24, 24A angeformt sind, um den Sensor zu halten. Im unteren Teil der Klemme befindet sich eine Klemmbacke 25, die gemäss Pfeil 26 nach oben bewegt werden kann, um den Sensor festzuklemmen. Ein Feststellknopf 27 ist an einem Feststellteil 28 angeordnet, der eine schiefe Ebene 29 aufweist, die auf eine ebenfalls schiefe Ebene 30 in der Klemmbacke 25 wirkt. Dabei wird der Sensor in der Klemme angeordnet und gegen die Greifer gedrückt.

Durch das Verstellen des Feststellteils 28, in der Zeichnung von rechts nach links, wird die Klemmbacke nach oben gedrückt. Wie aus Fig. 3 hervorgeht, weisen beide schiefen Ebenen 29 und 30 kleine Zähne 31 auf, wodurch die Bewegung beider schiefen Ebenen aufeinander gehemmt wird, so dass die Klemmbacke stets in der eingestellten Stellung verharrt. Der selbsthemmende Mechanismus wird durch eine Öffnungsbewegung gelöst, wobei die Haftreibung überwunden wird. Dieser Klemmmechanismus beansprucht wenig Platz, so dass dieser Halter wenig aufträgt und stört. Der Klemmmechanismus ist in einem Gehäuse 32 angeordnet.

Die Sensorklemme 22 kann an einem Halter montiert sein, der im Prinzip ähnlich demjenigen Halter gemäss EP-B-397 599 aufgebaut ist. Dabei sind sowohl der Aufbiss als auch der gekröpfte Indikatorstab dieselben oder ähnlich wie bei diesem europäischen Patent, während die federnde Röntgenfilm-Klemme durch die Sensorklemme 22 ersetzt ist.d.h., dass die Platte direkt nach dem Aufbiss montiert wird. Dabei ist in dieser Patentschrift offenbart, dass die Röntgenfilmklemme senkrecht zum Aufbiss angeordnet ist und der Röntgenfilm hochkant darin gehalten ist.

Allen Sensor-Klemmen gemäss der Erfindung ist gemeinsam, dass mit Ihnen die digitalen Sensoren ohne Probleme mit den hygienischen Beuteln oder sonstigen Schutzhüllen genau gehalten werden können und dass zwischen dem Sensor und der Röntgenröhre kein anderer Kunststoff vorhanden ist. Auch geht aus der Beschreibung klar hervor, dass Sensoren mit jeglichen Abmessungen gehalten werden können. Ferner ist aus der Beschreibung ersichtlich, dass die Sensoren immer gefasst werden und dass sie nicht in eine Klemme hineingepresst werden, da die Klemmbacke immer geöffnet werden kann, um den Sensor aufzunehmen, um ihn anschliessend zu fassen.

## Patentansprüche

1. Halter-Satz für digitale Sensoren für die Zahnmedizin, mit für jede Aufnahmeart wie periapikale Aufnahmen, für Seitenzähne und für Frontzähne ein Halter (32) für alle Sensoren-Formate, **dadurch gekennzeichnet, dass** der Halter des Satzes mindestens einen beweglichen Arm aufweist und die Sensorenklemme (22) Greifer (24, 24A) und davon beabstandet eine Klemmbacke (25) mit einer schiefen Ebene (30) aufweist, die mit einer schiefen Ebene (29) an einem FeststellTeil (28) am Halter zusammenarbeitet derart, dass durch Verschieben des Feststellteils in eine Richtung, die Klemmbacke gehoben wird, um den Sensor unabhängig von seinem Format gegen die Greifer (24, 24A) zu drücken, und wobei die Sensorenklemme an einer Indikatorstab (33) des Halters angeordnet ist.

2. Halter-Satz nach Anspruch 1, **dadurch gekennzeichnet, dass** die beiden schiefen Ebenen (29, 30) Zähne (31) aufweisen.

## Claims

1. Holder set for digital sensors for dentistry, comprising, for each type of radiograph such as periapical radiographs of lateral and of anterior teeth, a holder (32) for all sensor formats, **characterised in that** the holder of the set comprises at least one movable arm and the sensor clamp (22) has grippers (24, 24A) and at a distance therefrom a clamping jaw (25) provided with an inclined plane (30) that cooperates with an inclined plane (29) on a locking portion (28) of the holder in such a manner that by displacing the locking portion in one direction, the clamping jaw is lifted in order to push the sensor against the grippers (24, 24A) independently of its format, the sensor clamp being arranged on an indicator rod (33) of the holder.

2. Holder set according to claim 1, **characterised in that** the two inclined planes (29, 30) are provided with teeth (31).

## Revendications

1. Jeu de supports pour capteurs numériques pour la dentisterie, comprenant, pour chaque type de radiographie tel que radiographies péri-apicales de dents latérales et de dents antérieures, un porte-capteur (32) pour tous les formats de capteurs, **caractérisé en ce que** le porte-capteur de l'ensemble présente au moins un bras mobile et que la pince porte-capteur (22) comporte des griffes (24, 24A) et à l'écart de celles-ci une mâchoire de serrage (25) munie d'un plan oblique (30) qui coopère avec un plan oblique (29) sur un élément de blocage (28) du porte-capteur de telle manière qu'en déplaçant ledit élément de blocage dans une direction, la mâchoire de serrage est soulevée pour presser indépendamment de son format le capteur contre les griffes (24, 24A), la pince porte-capteur étant agencée sur une tige indicatrice (33) du porte-capteur.

2. Jeu de supports selon la revendication 1, **caractérisé en ce que** les deux plans obliques (29, 30) sont munis de dents (31).
